# EUROPÄISCHE PATENTANMELDUNG

(11) **EP 3 184 071 A1**
(43) Veröffentlichungstag der Anmeldung: **28.06.2017**
(21) Anmeldenummer: 15405076.9
(22) Anmeldetag: 22.12.2015
(51) Int. Cl.: A61B 90/00, A61B 34/20

(54) **VORRICHTUNG FÜR DIE INTRAOPERATIVE BILDGESTEUERTE NAVIGATION BEI CHIRURGISCHEN EINGRIFFEN IM BEREICH DER WIRBELSÄULE UND IM DARAN ANGRENZENDEN THORAX-, BECKEN- ODER KOPFBEREICH**

(71) Anmelder: SpineMind AG, 6370 Stans (CH)
(72) Erfinder: Jeszensky, Dezsö János, 8700 Küsnacht (CH); Fekete, Tamás Fülöp, 8008 Zürich (CH)
(74) Vertreter: EGLI-EUROPEAN PATENT ATTORNEYS

(57) **Zusammenfassung**

Die Erfindung betrifft eine Vorrichtung (1) für die intraoperative, bildgesteuerte Navigation bei chirurgischen Eingriffen im Bereich der Wirbelsäule und/oder im daran angrenzenden Thorax-, Becken- oder Kopfbereich, umfassend mehrere nicht-röntgenbasierte Detektionseinrichtungen (11-17), die jeweils dazu ausgebildet sind, verteilt um wenigstens ein zu operierendes Objekt (2) angeordnet zu werden und intraoperativ in Echtzeit Bild- und Lagedaten zu erfassen, die jeweils Informationen über zumindest die Aussenkontur wenigstens eines Teilbereichs des zu operierenden Objekts (2) sowie über die Lage des Teilbereichs relativ zur jeweiligen Detektionseinrichtung (11-17) umfassen. Ferner ist eine Positionsbestimmungseinrichtung (20) zur Positionsermittlung der jeweiligen Detektionseinrichtungen (11-17) relativ zu einem stationären Bezugssystem (30) sowie eine mit den Detektionseinrichtungen (11-17) und der Positionsbestimmungseinrichtung (20) wirkverbundene Datenverarbeitungseinrichtung (40) vorgesehen, die dazu ausgebildet ist, auf Basis der jeweiligen Bild- und Lagedaten der Detektionseinrichtungen (11-17) und der Lagedaten der Positionsbestimmungseinrichtung (20) ein auf das stationäre Bezugssystem (30) referenziertes, virtuelles, vorzugsweise dreidimensionales Echtzeit-Bild (41) des zu operierenden Objekts (2) zu erzeugen, das auf einer Bildanzeigeeinrichtung (50) dargestellt wird. Gemäss der Erfindung weist wenigstens eine der Detektionseinrichtungen (13-17) wenigstens ein Detektionsmittel (13.1-17.1) auf, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des Objekts (2) umfassen.

## Beschreibung

Die Erfindung betrifft eine Vorrichtung für die intraoperative bildgesteuerte Navigation bei chirurgischen Eingriffen gemäss dem Oberbegriff des Anspruchs 1 bzw. 16.

Aus dem Stand der Technik sind diverse Vorrichtungen und Verfahren für die intraoperative bildgesteuerte Navigation bekannt, die dem Chirurgen während einer Operation die Ausrichtung der verwendeten Instrumente oder Implantate relativ zum Patienten visualisieren. Das grundlegende Ziel dieser Navigationsvorrichtungen und -verfahren ist die Verbesserung der räumlichen Orientierung des Chirurgen während der Operation und damit letztlich die Erhöhung der Sicherheit des Patienten. Eine Vielzahl von Navigationssystemen basieren auf der als Registrierung bezeichneten Korrelierung bzw. Referenzierung von präoperativen Bilddaten, beispielsweise Computertomographie-(CT)- und/oder Magnetresonanztomographie-(MRT)-Bilddaten, mit dem intraoperativen Koordinatensystem des Patienten.

Grundsätzlich sind solche Registrierungsverfahren aus der medizinischen Bildverarbeitung bekannt. Dort dienen sie dazu, zwei oder mehrere Bilder derselben Szene, oder zumindest ähnlicher Szenen, bestmöglich in Übereinstimmung zu bringen, um aus ihrer Kombination bessere Erkenntnisse zu gewinnen. So werden z. B. MRT-Bilder, die Weichteilgewebe oder Gehirnstrukturen gut darstellen, mit CT-Bildern überlagert, um etwa die räumlichen Verhältnisse zwischen ossären Strukturen (CT-Bilder) und neutralen Elementen (MRT-Bilder) nachvollziehen zu können. Die zu registrierenden Bilder unterscheiden sich im Allgemeinen voneinander, etwa weil sie von unterschiedlichen Positionen, zu unterschiedlichen Zeitpunkten oder mit unterschiedlichen Verfahren (Modalitäten) aufgenommen wurden. Für die Registrierung wird in der Regel eines der Bilder als Referenzbild festgelegt, während die übrigen Objektbilder durch die Berechnung einer ausgleichenden Transformation, etwa auf Basis der Minimierung der mittlerem Fehlerquadrate, optimal an das Referenzbild angepasst werden.

In analoger Weise wird für den Registrierungsprozess bei der bildgesteuerten Navigation eine Transformation zwischen dem Koordinatensystem des Patienten und dem präoperativen Bilddatensatz durchgeführt, die auf der Identifizierung von Merkmalen bzw. Markern beruht, die sich sowohl in den präoperativen Bilddaten als auch in der realen Operationssituation am Patienten darstellen lassen. Das Ergebnis der Registrierung ist zumeist eine affine Transformation, welche eine Rotation, Translation und Skalierung der Bilddaten auf das Patienten-Koordinatensystem beinhaltet.

Für die Repräsentation des Patienten-Koordinatensystems werden einerseits sogenannte anatomische oder künstliche Landmarken und andererseits intraoperative Bildgebungsverfahren verwendet.

Die Registrierung über anatomische Landmarken erfordert die Auswahl geeigneter anatomischer Punkte am Patienten. In der Wirbelsäulenchirurgie werden die anatomischen Landmarken derart gewählt, dass sie etwa auf der Knochenoberfläche des Wirbels liegen. Um diese abzutasten, wird in der Regel die Knochenoberfläche der Wirbel freigelegt. Die gewählten Merkmale sollten das Operationsgebiet möglichst umschliessen, da dadurch die Genauigkeit der Registrierung steigt. Auf anatomischen Landmarken basierende Verfahren sind jedoch häufig mit einem grossen Zeitaufwand verbunden. So ist zwar beim Vorliegen einer normalen Anatomie das genaue Auffinden der Landmarken relativ genau möglich. Im Gegensatz dazu ist die Landmarkenerkennung bei Patienten mit wirbelsäulenpathologischen Veränderungen, etwa bei Tumoren, angeborenen, degenerativen oder traumatischen Veränderungen, deutlich erschwert und weniger genau. Oft resultiert dies in einer nur unbefriedigenden Navigationsgenauigkeit. Zudem ist dieses Verfahren sehr zeitaufwendig.

Eine Weiterentwicklung stellt die Verwendung künstlicher Landmarken und intraoperativer Bildgebungsverfahren dar, die die künstlichen Landmarken mitabbilden. Systeme, die auf künstlichen Landmarken basieren, bestehen in der Regel aus einem Lagesensor, einem Computer, einem Anzeigegerät und verschiedenen als künstliche Landmarken dienende Lokalisatoren. Die Lokalisatoren sind geeignete Referenzkörper, deren räumliche Lage durch den verwendeten Lagesensor gemessen wird. Für die Navigation werden diese Lokalisatoren z.B. in Form von Knochenschrauben am Patienten oder an Implantaten befestigt. Die Positionen künstlicher Landmarken können im Vergleich zu anatomischen Landmarken wesentlich exakter in den Bilddaten und in der Realität, d.h. in der realen Operationssituation am Patienten, vermessen werden, wodurch die Registriergenauigkeit gesteigert wird. Allerdings unterliegen auch künstliche Landmarken Störeinflüssen. Dies sind vornehmlich intraoperative Verschiebungen der Landmarken, bedingt durch intraoperative Manipulationen am Patienten oder auch versehentliches Verschieben während der Operation, was wiederum sehr zeitaufwändige Mehrfachregistrierungen notwendig macht. Mit der Erkenntnis, dass u.U. erst zu spät bemerkt werden könnte, dass sich künstliche Landmarken während der Operation verschoben haben, sinkt letztlich das grundsätzliche Vertrauen in die Navigation.

Demgegenüber ist beim Einsatz von intraoperativen Bildgebungsverfahren der Zeitaufwand deutlich geringer und die Registrierung des Patienten-Koordinatensystems mit den Bilddaten wesentlich reproduzierbarer. Vor allem die röntgenbasierte Registrierung oder eine Registrierung basierend auf 2D- oder 3D-Bildverstärkern bzw. intraoperativem CT ist hierbei sehr etabliert. Dabei wird beispielsweise ein präoperativ gewonnenes, CT-basiertes zwei- oder dreidimensionales Bild der Wirbelsäule mit einem intraoperativen zweidimensionalen Röntgenbild der Wirbelsäule über Software-Algorithmen fusioniert oder ein 3D-Datensatz wird intraoperativ erstellt (intraoperatives CT oder 3D-Bildverstärker), wodurch es insbesondere möglich ist, dass der Operateur etwa die Position der Operationsinstrumente und die Lage eingebrachter Implantate, etwa von Schrauben, intraoperativ in Echtzeit an einem dreidimensionalen Datensatz der Wirbelsäule verfolgen kann. Zudem ermöglicht die intraoperative Bildgebung mittels Röntgenstrahlen, noch während der Operation die Lage der implantierten Implantate zu kontrollieren und ggf. zu korrigieren, wobei dies durch wiederholte Bildgebung mittels Röntgenstrahlen zu höheren und meistens beträchtlichen Strahlendosen führt. Auch die Beurteilung und Anpassung des Alignements der Wirbelsäule ist mit intraoperativen Bildgebungsverfahren möglich. Jedoch sind viele Geräte für die intraoperative Bildgebung häufig sehr unflexibel und nehmen viel Platz ein, so dass sie auf den unmittelbaren Operationsbereich um den Patienten herum sehr einschränkend wirken. Zudem kommt - wie zuvor bereits erwähnt - bei jenen Bildgebungsverfahren, die auf Röntgenstrahlung basieren, als zusätzlicher Nachteil die Strahlenbelastung für den Patienten und das medizinische Personal hinzu.

Als Alternative hierzu sind aus dem Stand der Technik ferner Navigationsverfahren und Navigationsvorrichtungen bekannt, welche auf einer intraoperativen Ultraschall-Bildgebung basieren, wobei anstelle des oben beschriebenen intraoperativen röntgenbasierten Bilds ein intraoperativ genommener Ultraschall-Datensatz mit präoperativen Bilddaten fusioniert wird, um vorzugsweise in Echtzeit einen dreidimensionalen Datensatz der Wirbelsäule zu erhalten. Eine derartige Vorrichtung ist beispielsweise aus der internationalen Patentanmeldung WO 96/11624 bekannt. Dabei werden die Ultraschall-Datensätze von ausserhalb des Körpers in Richtung des Körperinneren aufgenommen, indem mit dem Ultraschallkopf jene Rückenpartie, die unmittelbar an den zu operierenden Knochenbereich angrenzt, von aussen, d.h. zum Beispiel auf der Rückenhaut aufsitzend, abgefahren wird.

Bei der klassischen röntgenbasierten wie auch bei der ultraschallbasierten intraoperativen Bildgebung wird das zu operierende Objekt jedoch entweder nur in Projektion oder aber nur ausschnittsweise in Form eines Konturbildes eines Teilbereichs abgebildet. Diese Projektionsbilder bzw. Teilbereichsbilder werden im Weiteren als Referenz genutzt, um mit präoperativen Bilddaten überlagert zu werden, auf deren Basis dann ein auf die intraoperativen Bilddaten referenziertes, virtuelles Echtzeit-Bild generiert wird. Dieses virtuelle Bild gibt jedoch nicht das zu operierende Objekt selbst in Echtzeit wieder, sondern nur dessen Echtzeit-Position. Die Projektionsbilder bzw. die Teilbereichskonturbilder sind allerdings - da sie gerade eben nur die Projektion bzw. nur einen Teilbereich wiedergeben - immanent nicht für die bildliche Wiedergabe des gesamten zu operierenden Objekts in Echtzeit bzw. der aktuellen intraoperativen Situation geeignet.

Aufgabe der vorliegenden Erfindung ist es daher, eine Vorrichtung für die intraoperative, bildgesteuerte Navigation bei chirurgischen Eingriffen im Bereich der Wirbelsäule und/oder im daran angrenzenden Thorax-, Becken- oder Kopfbereich anzugeben, die es erlaubt, ein virtuelles, vorzugsweise dreidimensionales Echtzeit-Bild des zu operierenden Objekts zu erzeugen, das zum Zwecke der Navigation auf ein stationäres Bezugssystem referenziert ist. Zudem soll die Vorrichtung den Operateur während der Operation nicht behindern und nicht-sensitiv gegen etwaige intraoperativ Störeinwirkungen sein, so dass stets eine hinreichende Navigationsgenauigkeit gewährleistet ist.

Diese Aufgabe wird durch eine Vorrichtung für die intraoperative, bildgesteuerte Navigation nach Anspruch 1 bzw. 16 gelöst. Vorteilhafte Ausgestaltungen der Erfindung sind Gegenstand der abhängigen Ansprüche.

Gemäss der Erfindung weist die Vorrichtung mehrere nicht-röntgenbasierte Detektionseinrichtungen, die jeweils dazu ausgebildet sind, verteilt um wenigstens ein zu operierendes Objekt im Bereich der Wirbelsäule und/oder im daran angrenzenden Thorax-, Becken- oder Kopfbereich angeordnet zu werden und intraoperativ in Echtzeit Bild- und Lagedaten zu erfassen, die jeweils Informationen über zumindest die Aussenkontur wenigstens eines Teilbereichs des zu operierenden Objekts sowie über die Lage des Teilbereichs relativ zur jeweiligen Detektionseinrichtung umfassen.

Als Aussenkontur des zu operierenden Knochenbereichs wird im Sinne der vorliegenden Erfindung die dreidimensionale Oberfläche des zu operierenden Objekts verstanden. Die Lage des Teilbereichs relativ zur jeweiligen Detektionseinrichtung ist im Sinne der vorliegenden Erfindung als die räumliche Anordnung der erfassten dreidimensionalen Oberfläche des zu operierenden Objekts relativ zur jeweiligen Detektionseinrichtung definiert, wobei die Anordnung/Lage einerseits den Abstand und andererseits die räumliche Orientierung/Ausrichtung in Bezug zur jeweiligen Detektionseinrichtung umfasst. Der Bezugspunkte "Detektionseinrichtung" ist so zu verstehen, dass damit vornehmlich der erfassende Teil der Detektionseinrichtung, d.h. das eigentliche Detektionsmittel, etwa die Sensorik, gemeint ist. Die Detektionseinrichtung ermittelt gemäss der Erfindung somit einerseits den Abstand und andererseits die Orientierung/Ausrichtung des jeweils von ihr erfassten dreidimensionalen Oberflächenbereichs des zu operierenden Objekts relativ zu sich selbst. Die entsprechenden Lagedaten umfassen daher Daten sowohl über den Abstand als auch die Ausrichtung der Aussenkonturdaten.

Das zu operierende Objekt kann grundsätzlich, aber nicht ausschliesslich Knochen-, andere Stützgewebe und/oder Weichteile (etwa pathologische Strukturen, Raumforderungen oder Tumore) umfassen. Im Bereich der Wirbelsäule kann das zu operierende Objekt insbesondere knöcherne und/oder knorpelige Teile der Wirbelsäule selbst umfassen, etwa einen oder mehrere Wirbel, oder einen oder mehrere Teile eines oder mehrerer Wirbel (Wirbelkörper, Querfortsatz, Dornfortsatz, Wirbelbogen, Ansatz für Rippen), das Kreuzbein (Os sacrum), das Steißbein (Os coccygis), und/oder eine oder mehrere Bandscheiben. Das zu operierende Objekt kann im Bereich der Halswirbelsäule ((Pars cervicalis), der Brustwirbelsäule (Pars thoracica) und/oder der Lendenwirbelsäule (Pars lumbalis) liegen. Weiterhin kann das zu operierendes Objekt ein oder mehrere Bänder oder Teile davon umfassen, etwa das vordere Längsband (Ligamentum longitudinale anterius), das hintere Längsband (Ligamentum longitudinale posterius), ein oder mehrere gelbe Bänder (Ligamenta flava), ein oder mehrere Zwischenquerfortsatzbänder (Ligamenta intertransversaria), ein oder mehrere Zwischendornfortsatzbänder (Ligamenta interspinalia) und/oder das Überdornfortsatzband (Ligamentum supraspinale). Das zu operierende Objekt kann auch im an die Wirbelsäule angrenzenden Beckenbereich liegen, also etwa einen angrenzenden Teil des knöchernen Beckens, insbesondere den Beckenring, betreffen. Ebenso kann das zu operierende Objekt im an die Wirbelsäule angrenzenden Thoraxbereich liegen, insbesondere einen oder mehrere Ansätze für Rippen an den Wirbel, eine oder mehrere Rippen(teile) oder sogar - bei massiven pathologischen Veränderungen - das Brustbein betreffen. Ebenso kann das zu operierende Objekt im an die Wirbelsäule angrenzenden Kopfbereich liegen. Dort kann es insbesondere den Bereich des sogenannten C0-Wirbels, also das Hinterhauptbein (Os occipitale) bzw. das Occiput umfassen. Auch kann das zu operierende Objekt sonstiges Weichgewebe, insbesondere nervale Elemente oder pathologische Strukturen, im Bereich der Wirbelsäule und/oder im daran angrenzenden Thorax-, Becken- oder Kopfbereich umfassen.

Gemäss der Erfindung sind die Detektionseinrichtungen jeweils dazu ausgebildet, Bilddaten zu erfassen, die jeweils Informationen über zumindest die Aussenkontur des zu operierenden Objekts bzw. eines Teilbereichs davon umfassen. Je nach Art bzw. Detektionsprinzip der Detektionseinrichtung und je nach Aufbau des zu operierenden Objekts kann eine oder mehrere dieser nicht-röntgenbasierten Detektionseinrichtungen mitunter auch dazu ausbildet sein, neben der Aussenkontur des zu operierenden Objekts auch bildgebende Informationen über die innere Struktur des zu operierenden Objekts zu erfassen. So können etwa auf Ultraschall oder Terahertzstrahlung basierende Detektionseinrichtungen in der Lage sein, je nach Struktur, Aufbau und Gewebe/Material des zu operierenden Objekts über die Aussenkontur hinaus mit einer materialabhängigen Eindringtiefe oder sogar Durchdringung Informationen über die innere Struktur des zu operierenden Objekts zu liefern.

Die erfindungsgemässe Vorrichtung umfasst im Weiteren eine Positionsbestimmungseinrichtung, die dazu ausgebildet ist, in Echtzeit Daten zur jeweiligen Position der Detektionseinrichtungen relativ zu einem stationären Bezugssystem zu ermitteln, vorzugsweise zu einem relativ zum Operationssaal stationären Bezugssystem. Auch hier ist der Bezugspunkte "Detektionseinrichtung" im Hinblick auf die relative Lage zum stationären Bezugssystem so zu verstehen, dass damit vornehmlich der erfassende Teil der Detektionseinrichtung, d.h. das eigentliche Detektionsmittel, etwa die Sensorik, gemeint ist. Mit der Positionsbestimmungseinrichtung als letztes Glied in der Kette zwischen dem zu operierenden Objekt und dem stationären Bezugssystem wird insgesamt gewährleistet, dass die erfassten Aussenkonturdaten der dreidimensionalen Oberfläche jedes Teilbereichs des zu operierenden Objekts über die Lagedaten der Detektionseinrichtungen relativ zum jeweils erfassten Teilbereich und im Weiteren über die Lagedaten der Detektionseinrichtungen relativ zum stationären Bezugsystem räumlich auf das stationären Bezugsystems referenziert werden können. Somit stehen insgesamt Information darüber zur Verfügung, wie die einzelnen erfassten Teilbereiche der dreidimensionalen Oberfläche des zu operierenden Objekts relativ zum stationären Bezugssystem orientiert sind.

Erfindungsgemäss ist ferner eine Datenverarbeitungseinrichtung jeweils mit den mehreren Detektionseinrichtungen und der Positionsbestimmungseinrichtung wirkverbunden. Die Datenverarbeitungseinrichtung ist dazu ausgebildet, ein auf das stationäre Bezugssystem referenziertes, virtuelles, vorzugsweise dreidimensionales Echtzeit-Bild des zu operierenden Objekts zu erzeugen, und zwar auf Basis der jeweiligen Bild- und Lagedaten der Detektionseinrichtungen sowie auf Basis der jeweiligen Positionsdaten der Positionsbestimmungseinrichtung.

Zur Darstellung des virtuellen Echtzeit-Bildes ist gemäss der Erfindung ferner eine Bildanzeigeeinrichtung vorgesehen, die mit der Verarbeitungseinrichtung wirkverbunden ist.

Gemäss der Erfindung weist ausserdem wenigstens eine der mehreren Detektionseinrichtungen wenigstens ein Detektionsmittel auf, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen.

Dabei bedeutet "dem Körperinneren zugewandt", dass die entsprechende Aussenkontur der Wirbelsäule oder des an die Wirbelsäule angrenzenden Thorax-, Becken- oder Kopfbereichs während der Operation intrakorporal liegt. Somit kann "dem Körperinneren zugewandt" nicht nur "medial" oder "profund", d.h. zur Mitte des Körpers hin, insbesondere einen "ventral" gelegenen Teilbereich umfassen, sondern auch seitlich, seitlich schräg nach hinten, seitlich schräg nach vorn, hinten schräg nach oben, hinten schräg nach unten, vorn schräg nach oben, vorn schräg nach unten, seitlich schräg nach oben oder seitlich schräg nach unten weisend. Diese Orts- und Richtungsangaben beziehen sich rein definitionshalber auf einen aufrecht stehenden Patienten. Der entsprechende Teilbereich kann während der Operation freipräpariert, teilweise freipräpariert oder vollständig von Gewebe umgeben sein.

Es wurde in erfindungsgemässer Weise erkannt, dass durch die Verwendung mehrerer Detektionseinrichtungen, die um die Operationsstelle herum verteilt sind und von denen im Speziellen wenigstens eine im Inneren des Körpers angeordnet ist, anstelle nur eines begrenzten Teilausschnitts bzw. einer Projektion ein wesentlich umfassenderes Bild des zu operierenden Objekts aus verschiedenen Blickwinkeln erzeugt werden kann, das insbesondere Informationen über zumindest die Aussenkontur wenigstens eines dem Körperinneren zugewandten Teilbereichs des zu operierenden Objekts enthält. Im Idealfall kann sogar ein virtuelles, vorzugsweise dreidimensionales Echtzeit-Rundumbild bzw. eine vollständige Echtzeit-3D-Rekonstruktion der Aussenkontur des zu operierenden Objekts erzeugt werden, was in erfindungswesentlicher Weise die Navigationsgenauigkeit und das Sicherheitsgefühl für den Operateur und letztlich die Sicherheit des Patienten deutlich verbessert.

Insbesondere beruht das mit der erfindungsgemässen Vorrichtung erzeugte virtuelle Echtzeit-Bild auf tatsächlichen Echtzeit-Bilddaten, die nicht nur die Nachverfolgung der Echtzeit-Position des Operationsbereichs relativ zum stationären Bezugssystem erlauben, sondern auch in Echtzeit die tatsächliche Aussenkontur des zu operierenden Objekts quasi in situ bildlich wiedergeben. In vorteilhafter Weise können daher mit der erfindungsgemässen Vorrichtung - im Gegensatz zu Verfahren und Vorrichtungen, bei denen das virtuelle Bild der Operationsstelle abgesehen von den Positionsdaten ausschliesslich auf präoperativen oder während des ersten Teils der Operation, d.h. während der Zugangsphase, genommenen Daten beruhen - intraoperative Veränderungen an der Aussenkontur des zu operierenden Objekts und in der Umgebung der Operationsstelle in Echtzeit bildlich erfasst werden. So werden etwa im Operationsbereich intraoperativ vorgenommene Knochen- oder übrige Gewebeabtragungen, eingebrachte Schrauben, in Ansatz gebrachte Operationswerkzeuge, Stellungskorrekturen/Umstellungen des Alignements oder dergleichen in situ bildlich erfasst, wodurch im Weiteren die Navigationsgenauigkeit und das Sicherheitsgefühl für den Operateur in erfindungswesentlicher Weise gesteigert werden.

Selbstverständlich kann nicht nur eine Detektionseinrichtung dazu ausgebildet sein, um im Inneren des Körpers angeordnet zu werden bzw. intraoperativ angeordnet zu sein, sondern wenigstens zwei, wenigstens drei, wenigstens vier, wenigstens fünf, wenigstens sechs, wenigstens sieben, wenigstens acht, wenigstens neun, wenigstens zehn, wenigstens elf, wenigstens zwölf oder mehr; insbesondere zwei, drei, vier, fünf, sechs, sieben, acht, neun, zehn, elf, zwölf der mehreren Detektionseinrichtungen.

Die Anzahl und Anordnung der Gesamtheit der mehreren Detektionseinrichtungen kann sich nach der Grösse und Lage des zu operierenden Objekts richten. Denkbar ist beispielsweise, dass nur ein kleiner Teil/Abschnitt der Wirbelsäule von der Operation betroffen ist. Hierfür kann bereits eine geringe Anzahl an Detektionseinrichtungen ausreichend sein, die in einem eng begrenzten Bereich um die Operationsstelle herum angeordnet sind. In einem anderen Fall kann die Operation einen wesentlichen grösseren Bereich betreffen, der sich insbesondere über die gesamte Länge der Wirbelsäule erstrecken kann, so dass eine grössere Anzahl von Detektionseinrichtungen notwendig ist, die über die gesamte Länge des Operationsbereichs entlang der Wirbelsäule verteilt angeordnet sind.

Ebenso kann die Art der Detektionseinrichtung je nach Position, insbesondere abhängig von der Anordnung innerhalb oder ausserhalb des Körpers, für die mehreren Detektionseinrichtungen unterschiedlich sein. Denkbar ist beispielsweise, dass eine Detektionseinrichtung, die innerhalb des Körpers angeordnet ist, eine ultraschallbasierte Detektionseinrichtung ist, während eine Detektionseinrichtung der gleichen Vorrichtung, die ausserhalb des Körpers angeordnet ist, ein optischer Konturscanner ist. Durch die Verwendung mehrerer Detektionseinrichtungen unterschiedlicher Art können in vorteilhafter Weise mehr und detailliertere Informationen über das zu operierende Objekt gewonnen werden.

Denkbar ist ferner, dass sich die erfassten Teilbereiche des zu operierenden Objekts, die jeweils unterschiedlichen Detektionseinrichtungen zugeordnet sind, teilweise überlappen, d.h. dass sich die Detektionsfelder unterschiedlicher Detektionseinrichtungen teilweise überlappen.

Die Erzeugung des virtuellen Echtzeit-Bildes kann so erfolgen, dass die einzelnen von den Detektionseinrichtungen erfassten Bilddatensätze, die jeweils wenigstens die Aussenkontur eines Teilbereichs des zu operierenden Objekts wiedergeben, von der Datenverarbeitungseinrichtung zu einem Gesamtbild zumindest der Aussenkontur des zu operierenden Objekts zusammengesetzt werden. Hierfür werden einerseits die von der Detektionseinrichtung bestimmten Daten zur Lage, d.h. Orientierung und Abstand, des entsprechenden Teilbereichs relativ zur jeweiligen Detektionseinrichtung und andererseits die von der Positionsbestimmungseinrichtung erfassten Postionsdaten der jeweiligen Detektionseinrichtung relativ zum stationären Bezugssystem verwendet, um mithilfe dieser Informationen aus den Bilddatensätzen der einzelnen Teilbereiche das Gesamtbild des zu operierenden Objekts zusammenzusetzen. Hierzu kann in der Detektionseinrichtung eine spezielle Bildregistrierungs-, Bildgenerierung- und/oder Bildbearbeitungssoftware zum Einsatz kommen, die aus den Bilddatensätzen den zugeordneten Lage- bzw. Positionsdaten ein virtuelles Bild zumindest der Aussenkontur des zu operierenden Objekts rekonstruiert. Zum räumlich exakten Zusammensetzen der einzelnen Bilddatensätze zu einem Gesamtbild kann die Datenverarbeitungseinrichtung insbesondere dazu ausgebildet sein, spezielle Bildbereiche zu identifizieren, die anatomisch markante Merkmale des zu operierenden Objekts aufweisen, beispielsweise Dornfortsätze, Querfortsätze, Gelenkfortsätze, Rippen, Rippenansätze oder andere markante Merkmale im Wirbelsäulen-, Becken- Thorax oder Occiput-Bereich. Auch Gefässe können als anatomisch markante Merkmale genutzt werden, die sich im Bereich des zu operierenden Objekts befinden, beispielsweise die Arteria vertebralis, die jeweils durch das Foramen transversarium läuft. Vorzugsweise kann die Datenverarbeitungseinrichtung dazu ausgebildet sein, gleiche anatomisch markante Objekte in den Bilddatensätzen, die von verschiedenen Detektionseinrichtungen stammen, zu suchen und anhand der Position und Orientierung dieser anatomisch markanten Objekte die Bilddatensätze der verschiedenen Detektionseinrichtungen räumlich einander zuzuordnen bzw. in der richtigen Weise räumlich zueinander anzuordnen.

Für die Erzeugung des virtuellen Echtzeit-Bilds können insbesondere grundsätzlich bekannte Verfahren der Bildregistrierung zum Einsatz kommen. Beispielsweise kann das Registrierungsverfahren folgende Schritte umfassen: In einem ersten Schritt erfolgt die sogenannte Merkmalsextraktion: Aus den zu registrierenden Bilddatensätzen werden anatomisch markante Merkmale des zu operierenden Objekts, wie z. B. Dorn- oder Querforstätze oder dergleichen, manuell oder automatisch detektiert. In einem zweiten Schritt, der sogenannten Merkmalsanpassung, wird die Korrespondenz der extrahierten Merkmalspunkte hergestellt. Hierzu kann die Detektionseinrichtung beispielsweise eine Funktion verwenden, die ein qualitatives Mass für Übereinstimmungen in den Echtzeit-Bilddatensätzen der verschiedenen Detektionseinrichtungen angibt, welches dann in einem Optimierungsverfahren zu minimieren oder maximieren ist. In einem dritten Schritt erfolgt die Transformationsberechnung, in der ein geeigneter Transformationstyp, z. B. ein affiner Transformationstyp, ein projektiver Transformationstyp oder dergleichen, gewählt und die Transformationsparameter berechnet werden. Schliesslich erfolgt in einem vierten Schritt die eigentliche Transformation. Dazu werden die Bilddatensätzen mit der im vorherigen Schritt berechneten Umbildung transformiert, wobei mitunter auch Interpolationstechniken zum Einsatz kommen können.

Nach einer ersten vorteilhaften Ausgestaltung der Erfindung können neben den Echtzeit-Bilddaten präoperativ oder während der Zugangsphase gewonnene Bilddaten des zu operierenden Objekts mit in die Erzeugung des virtuellen Echtzeit-Bildes integriert werden. Daher kann es insbesondere vorgesehen sein, dass die Datenverarbeitungseinrichtung dazu ausgebildet ist, präoperativ oder während der Zugangsphase gewonnene Bilddaten des zu operierenden Objekts mit den Bilddaten der Detektionseinrichtungen zu überlagern, um auf Basis der mit den Bilddaten der Detektionseinrichtungen überlagerten, präoperativ oder während der Zugangsphase gewonnenen Bilddaten das virtuelle auf das stationäre Bezugssystem referenzierte Echtzeit-Bild des zu operierenden Objekts zu erzeugen. Auch hierbei können wiederum grundsätzlich bekannte Bildregistrierungsverfahren zum Einsatz kommen. Durch die zusätzliche Einbindung präoperativ gewonnener Bilddaten können in vorteilhafter Weise mehr und detailliertere Informationen über den zu operierenden Objekts gewonnen werden.

Grundsätzlich ist aber auch denkbar, dass das virtuelle Echtzeit-Bild im Hinblick auf die graphischen Wiedergabe des zu operierenden Objekts nur auf Basis der mit den Aussenkonturdaten überlagerten präoperativ gewonnenen Bilddaten erzeugt wird, während die intraoperativ gewonnenen Bilddaten, Lagedaten und Positionsdaten nur zur Echt-Referenzierung dieses virtuellen Echtzeit-Bildes auf das stationäre Bezugssystem verwendet werden. Insoweit wird nach einem unabhängigen Gedanken der Erfindung eine weitere Vorrichtung für die intraoperative, bildgesteuerte Navigation bei chirurgischen Eingriffen im Bereich der Wirbelsäule und/oder im daran angrenzenden Thorax-, Becken- oder Kopfbereich vorgeschlagen, die wenigstens eine vorzugsweise nicht-röntgenbasierte Detektionseinrichtung umfasst, die dazu ausgebildet ist, intraoperativ in Echtzeit Bild- und Lagedaten zu erfassen, die Informationen über zumindest die Aussenkontur wenigstens eines Teilbereichs des zu operierenden Objekts sowie über die Lage des Teilbereichs relativ zur Detektionseinrichtung umfassen. Die Vorrichtung umfasst darüber hinaus eine Positionsbestimmungseinrichtung, dazu ausgebildet ist, in Echtzeit Daten zur Position der wenigstens einen Detektionseinrichtung relativ zu einem stationären Bezugssystem zu ermitteln. Ferner umfasst die Vorrichtung eine mit der wenigstens einen Detektionseinrichtung und der Positionsbestimmungseinrichtung wirkverbundene Datenverarbeitungseinrichtung, die dazu ausgebildet ist, präoperativ oder während der Zugangsphase gewonnene Bilddaten des zu operierenden Objekts mit den Bilddaten der wenigstens einen Detektionseinrichtung zu überlagern, um auf Basis der mit den Bilddaten der Detektionseinrichtung überlagerten, präoperativ oder während der Zugangsphase gewonnenen Bilddaten das virtuelle auf das stationäre Bezugssystem referenzierte Echtzeit-Bild des zu operierenden Objekts zu erzeugen. Auch hierbei können wiederum grundsätzlich bekannte Bildregistrierungsverfahren zum Einsatz kommen. Zur Darstellung des virtuellen Echtzeit-Bildes ist im Weiteren eine mit der Datenverarbeitungseinrichtung wirkverbundene Bildanzeigeeinrichtung vorgesehen. Auch bei dieser Vorrichtung weist die wenigstens eine Detektionseinrichtung wenigstens ein Detektionsmittel auf, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen. Insoweit dient die wenigstens eine Detektionseinrichtung vornehmlich der Nachverfolgung der Echtzeit-Position des Operationsbereichs relativ zum stationären Bezugssystem, indem sie wenigstens einen dem Körperinneren zugewandten Teilbereich des zu operierenden Objekts erfasst und als Referenzbereich zur Überlagerung mit den präoperativ oder während der Zugangsphase gewonnenen Bilddaten.

Sowohl die Vorrichtung nach Anspruch 1 als auch die Vorrichtung nach Anspruch 16 beruhen somit auf dem gleichen Erfindungsgedanken, nämlich dass (die) wenigstens eine Detektionseinrichtung bzw. ein Detektionsmittel dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen. In erfindungsgemässer Weise wurde erkannt, dass die Anordnung der Detektionseinrichtung bzw. des Detektionsmittels im Körperinneren die gesamte Navigation unempfindlich gegen intraoperative Störeinwirkungen macht, so dass stets eine hinreichende Navigationsgenauigkeit gewährleistet ist. Durch die Anordnung im Inneren des Körpers besteht insbesondere eine wenn nur sehr geringe Gefahr, dass das Detektionsfeld der Detektionseinrichtung vom Operateur gestört wird. Umgekehrt beeinträchtigt die wenigstens eine im Körperinneren anordenbare Detektionseinrichtung in vorteilhafter Weise auch nicht die Zugänglichkeit des Operationsbereichs für den Operateur. Des Weiteren erlauben es Detektionsmittel im Inneren des Körpers, andere bildgebende Modalitäten zu benutzen als beispielsweise Röntgenstrahlung, etwa wie beispielsweise Ultraschall. Ultraschall hat insbesondere im Bereich der Halswirbelsäule grosse Vorteile. So kann durch beispielsweise mit entsprechend angepassten Geräten, wie z.B. Geräten für die transösophageale Echokardiographie, die Halswirbelsäule von vorne abgebildet werden, wodurch wichtige Strukturen identifiziert werden können (etwa die Arteria vertebralis oder Nervenwurzeln). Gleichzeitig können die Bewegungen der Vertebrae (Wirbel), die aufgrund der grossen Mobilität der Halswirbelsäule durch chirurgische Manipulation oder sonstige Manöver verursacht werden, kontinuierlich kompensiert werden. Zudem erlauben Detektionsmittel im Inneren des Körpers, andere Aspekte des zu operierenden Objektes darzustellen, die nicht bereits über den Zugangsweg zum zu operierenden Objekt einsehbar sind. Dies führt im Weiteren zu einer Erhöhung der Genauigkeit.

Von Bedeutung ist ebenso, dass die im Körperinneren anordenbare Detektionseinrichtung aufgrund der aktiven Positionsbestimmung mit Hilfe der Positionsbestimmungseinrichtung nicht fixiert werden muss. Vielmehr kann sie sich frei bewegen, sodass die Navigation unempfindlich gegenüber intraoperativen Störeinwirkungen ist, etwa Manipulation am Patient durch den Operateur, Umlagerungen, Atem- oder Darmbewegungen etc. Denn die Positionsbestimmungseinrichtung erfasst gemäss der Erfindung kontinuierlich einerseits die jeweilige Lage der Detektionseinrichtungen relativ zum stationären Bezugssystem. Andererseits überwacht die Detektionseinrichtungen selbst in Echtzeit permanent ihre Lage relativ zu dem von ihnen erfassten Teilbereich der Operationsstelle. Hierdurch wird ermöglicht, die erfassten Aussenkonturdaten und folglich das virtuelle Echtzeit-Bild auf das stationäre Bezugssystem in Echtzeit zu referenzieren.

Nach einer weiteren vorteilhaften Ausgestaltung der Erfindung ist die wenigstens eine im Inneren des Körpers anordenbare Detektionseinrichtung eine ultraschallbasierte Detektionseinrichtung. Ultraschallbasierte Detektionseinrichtungen sind in besondere Weise für die Bildgebung in Echtzeit geeignet. Ferner erlauben ultraschallbasierte Detektionseinrichtungen, die Aussenkontur des zu operierenden Objekts durch Weichgewebe hindurch zu erfassen, also etwa durch Fettgewebe, Muskelgewebe und/oder Bindegewebe. Daher eignen sie sich insbesondere zur Bilderfassung solcher Teilbereiche des zu operierenden Objekts, die intraoperativ nicht freipräpariert werden. Darüber hinaus sind ultraschallbasierte Detektionseinrichtungen mitunter dazu ausgebildet, eine Abstands- und Lagemessung zwischen Detektionseinrichtung und erfasstem Objekt sowie eine räumliche Vermessung des zu operierenden Objekts zu ermöglichen, so dass die Lage des erfassten Teilbereichs relativ zur Detektionseinrichtung sehr genau in Echtzeit erfasst werden kann. Ultraschallbasierte Detektionseinrichtungen erlauben zudem Doppler-sonografische Messungen von Flüssigkeitsströmungen. So können z.B. wichtige Blutgefässe als Gefahrenzonen oder anatomische Landmarken identifiziert werden. Auch können Flüssigkeitsströmungen im Liquorraum detektiert werden, die zwar zumeist minimal, aber dennoch im Hinblick auf die Operation von grösster Relevanz sind.

Denkbar ist auch, dass die wenigstens eine im Inneren des Körpers anordenbare Detektionseinrichtung eine auf Terahertzstrahlung basierte Detektionseinrichtung ist. Der große Brechungsindex von organischem Gewebe im Terahertz-Spektrum erlaubt sehr kontrastreiche Aufnahmen und kann konventionelle Aufnahmetechniken ergänzen. Die Strahlung ist nicht-ionisierend und kann gefahrlos für medizinische und biologische Anwendungen eingesetzt werden. Terahertzstrahlung vermag u.U. sogar je nach Material/Gewebe Informationen über die innere Struktur des zu operierenden Objekts zu liefern. Information über die innere Struktur können beispielsweise helfen, zwischen gesunden Zellen und Tumorzellen zu unterscheiden

Im Speziellen kann es nach einer weiteren Ausgestaltung der Erfindung vorgesehen sein, dass die wenigstens eine im Inneren des Körpers anordenbare Detektionseinrichtung dazu ausgebbildet ist, über eine präformierte Körperhöhle, insbesondere die Nase, den Mund, den Rachenraum, die Speiseröhre, die Luftröhre, den Magen-Darmtrakt, die Harnblase, oder über ein Blutgefäss, in den Körper eingebracht zu werden. Für solche Applikationen eignen sich insbesondere endoskopische Detektionseinrichtungen für die Endosonografie, etwa für die gastroenterologische Endosonografie. Die Lage/Position letztgenannter Detektionsrichtungen für die gastroenterologische Endosonografie kann entweder direkt über das Endoskop oder etwa mittels elektromagnetischer Wellen (Funk) nach aussen erfolgen. Für die Positionsbestimmung können insbesondere Lagesensoren in Betracht kommen, die mitunter in der Lage sind, am Endoskop auftretende Beschleunigung zu messen, um daraus auf die Lage und Position des Detektionsmittels zu schliessen.

Ferner kann es vorgesehen sein, dass die wenigstens eine im Inneren des Körpers anordenbare Detektionseinrichtung dazu ausgebbildet ist, operativ in ein Gewebe, beispielsweise in paravertebrales Muskelgewebe oder Muskelgewebe des Gesässmuskels, und/oder in ein Fettgewebe, beispielsweise den Epiduralraum, eingebracht zu werden. Für solche Applikationen eignen sich neben den oben beschriebenen, konventionellen Endosonografie-Sonden insbesondere sogenannte sonografische Minisonden-Systeme, die etwa durch einen Biopsiekanal geschoben werden können und eine wesentlich dünnere Sonde als bei der konventionellen Endosonografie aufweisen.

Analog zu der wenigstens einen im Inneren des Körpers anordenbaren Detektionseinrichtung kann wenigstens eine der mehreren Detektionseinrichtungen dazu ausgebildet sein, ausserhalb des Körpers angeordnet zu werden, um Bilddaten zu erfassen, die Informationen über zumindest die Aussenkontur eines dem Körperäusseren zugewandten Teilbereichs des zu operierenden Objekts umfassen.

In gleicher Weise kann es ferner vorgesehen sein, dass die wenigstens eine ausserhalb des Körpers anordenbare Detektionseinrichtung eine ultraschallbasierte Detektionseinrichtung oder eine auf Terahertzstrahlung basierte Detektionseinrichtung ist. Denkbar ist aber auch, dass die wenigstens eine ausserhalb des Körpers anordenbare Detektionseinrichtung als ein optischer Konturscanner ausgebildet ist.

Als Positionsbestimmungseinrichtungen kommen beispielsweise elektromagnetische, insbesondere optische Positionsbestimmungseinrichtungen in Betracht, bei denen an den Detektionseinrichtungen jeweils ein passiver Positionsmarker oder ein aktiver Positionssender angeordnet ist, der über eine entfernt angeordnete Positionsmarker-Erkennungseinrichtung oder eine entfernt angeordnete Empfängereinrichtung, die von dem Positionssender ausgesandte Signale empfängt, überwacht wird. Solche Positionsbestimmungseinrichtungen sind grundsätzlich aus dem Stand der Technik bekannt, beispielsweise die für die Spinalchirurgie unter dem Markennamen "StealthStation" vertriebene Positionsbestimmungseinrichtung von Medtronic.

Um dem Operateur - wie zuvor erläutert - auch Echtzeit-Informationen zu etwaigen Operationswerkzeugen und deren Position und Orientierung relativ zur Operationsstelle intraoperativ zur Verfügung zu stellen, kann es nach einer weiteren Ausgestaltung der Erfindung vorgesehen sein, dass die Vorrichtung zum einen wenigstens ein Operationswerkzeug umfasst, und dass zum anderen die Positionsbestimmungseinrichtung ferner zur Ermittlung der Lage des wenigstens einen Operationswerkzeuges relativ zum stationären Bezugsystem ausgebildet ist und die Verarbeitungseinheit ferner zum Einblenden eines Indikators in das virtuelle Echtzeit-Bild ausgebildet ist, der das wenigstens eine Operationswerkzeug und dessen Lage relativ zum stationären Bezugsystem repräsentiert.

Darüber hinaus kann es bei einer weiteren vorteilhaften Ausgestaltung der Erfindung vorgesehen sein, dass wenigstens eine der Detektionseinrichtungen an einem Operationswerkzeug anordenbar ist bzw. angeordnet ist. Hierzu kann die erfindungsgemässe Vorrichtung ferner ein Operationswerkzeug umfassen, das für die Aufnahme wenigstens einer Detektionseinrichtung ausgebildet ist bzw. an dem wenigstens eine Detektionseinrichtung angeordnet ist.

Um die Funktionalität der Vorrichtung gegen etwaige äussere Störeinflüsse abzusichern und somit das Sicherheitsgefühl für den Operateur konstant aufrecht zu erhalten, kann es nach einer weiteren vorteilhaften Ausgestaltung der Erfindung vorgesehen sein, dass die Datenverarbeitungseinrichtung dazu ausgebildet ist, bei einem temporären Ausfall der Bilddaten-, der Lagedaten und/oder der Positionsdatenermittlung wenigstens einer der Detektionseinrichtungen den von dieser Detektionseinrichtung erfassten Teilbereich des zu operierenden Objekts in dem virtuellen Echtzeit-Bild auf Basis der zu einem früheren Zeitpunkt von dieser Detektionseinrichtung bei funktionierender Lagedaten- und Positionsdatenermittlung erfassten Bilddaten zu erzeugen, wobei die zu einem früheren Zeitpunkt erfassten Bilddaten des entsprechenden Teilbereichs in Echtzeit an die aktuelle Lage der von den übrigen Detektionseinrichtungen erfassten Teilbereiche anpasst werden.

Für den Fall, dass lediglich die Lagedaten- und/oder Positionsdatenermittlung für eine oder mehrere der Detektionseinrichtungen temporär ausfällt, nicht jedoch die Bilderfassung der betroffene(n) Detektionseinrichtung(en), kann es nach einer weiteren Ausgestaltung der Erfindung vorgesehen sein, den von dieser Detektionseinrichtung erfassten Teilbereich des zu operierenden Objekts in dem virtuellen Echtzeit-Bild zwar auf Basis der aktuell erfassten Bilddaten zu erzeugen, jedoch die aktuell erfassten Bilddaten des entsprechenden Teilbereichs im Hinblick auf die Lage relativ zum stationären Bezugssystem in Echtzeit an die aktuelle Lage der von den übrigen Detektionseinrichtungen erfassten Teilbereiche anzupassen.

Ferner kann es vorgesehen sein, dass die Detektionseinrichtungen dazu ausgebildet sind, miteinander zu kommunizieren, insbesondere Informationen über ihre jeweilige Position und/oder Lage, vorzugsweise relativ zum stationären Bezugssystem, auszutauschen. Darüber hinaus kann es vorgesehen sein, dass wenigstens eine der mehreren Detektionseinrichtungen, insbesondere wenigstens ein Detektionsmittel wenigstens einer der Detektionseinrichtungen, dazu ausgebildet sind, die Position und/oder Lage wenigstens einer anderen Detektionseinrichtung bzw. die Position und/oder Lage des jeweiligen Detektionsmittels der wenigstens einen anderen Detektionseinrichtung relativ zu sich und damit über die Positionsbestimmungseinrichtung relativ zum stationären Bezugssystem zu ermitteln. Hierdurch kann die Navigationsgenauigkeit im Weiteren deutlich verbessert werden. Denkbar ist beispielsweise, dass eine der mehreren Detektionseinrichtungen eine Ultraschall-Detektionseinrichtung ist, die nicht nur dazu ausgebildet ist, Bild- und Lagedaten über wenigstens einen Teilbereich des zu operierende Objekts zu erfassen, sondern auch wenigstens Lage- und Postionsdaten, vorzugsweise auch Bilddaten, zu wenigstens einer anderen Detektionseinrichtung relativ zu sich zu erfassen.

Um zusätzlich weitere Informationen über die Aussenkontur und insbesondere die innere Struktur des zu operierenden Objekts zu erhalten, kann es nach einer weiteren Ausgestaltung der Erfindung vorgesehen sein, dass die Vorrichtung ferner wenigstens eine röntgenbasierte Detektionseinrichtung aufweist, die dazu ausgebildet ist, intraoperativ in Echtzeit Bilddaten zu erfassen, die Informationen über die Aussenkontur und innere Struktur wenigstens eines Teilbereichs des zu operierenden Objekts umfassen, wobei die Datenverarbeitungseinrichtung mit der röntgenbasierten Detektionseinrichtung wirkverbunden ist und dazu ausgebildet ist, die Bilddaten der röntgenbasierten Detektionseinrichtung mit den Bilddaten der nicht-röntgenbasierten Detektionseinrichtungen zu überlagern, um auf Basis der miteinander überlagerten Bilddaten das virtuelle auf das stationäre Bezugssystem referenzierte Echtzeit-Bild des zu operierenden Objekts zu erzeugen, das Informationen über die Aussenkontur und innere Struktur des zu operierenden Objekts umfasst. Denkbar ist auch, dass zusätzlich präoperativ oder während der Zugangsphase gewonnene Bilddaten - wie weiter oben beschrieben - mit zuvor genannten intraoperativen Daten der röntgenbasierten Detektionseinrichtung und nicht-röntgenbasierten Detektionseinrichtungen überlagert werden.

Nach einer weiteren Ausgestaltung der Erfindung kann es auch vorgesehen sein, dass die Vorrichtung ferner wenigstens eine röntgenbasierte Detektionseinrichtung aufweist, die dazu ausgebildet ist, intraoperativ in Echtzeit Bild- und Lagedaten zu erfassen, die Informationen über die Aussenkontur und innere Struktur wenigstens eines Teilbereichs des zu operierenden Objekts sowie über die Lage des Teilbereichs relativ zur röntgenbasierten Detektionseinrichtung umfassen, wobei die Positionsbestimmungseinrichtung ferner dazu ausgebildet ist, in Echtzeit Daten zur Position der röntgenbasierten Detektionseinrichtung relativ zum stationären Bezugssystem zu ermitteln, und wobei die Datenverarbeitungseinrichtung mit der röntgenbasierten Detektionseinrichtung wirkverbunden ist und dazu ausgebildet ist, auf Basis der jeweiligen Bild- und Lagedaten der nicht-röntgenbasierten Detektionseinrichtungen, der Bild- und Lagedaten der röntgenbasierten Detektionseinrichtung und der jeweiligen Positionsdaten der Positionsbestimmungseinrichtung ein auf das stationäre Bezugssystem referenziertes, virtuelles, vorzugsweise dreidimensionales Echtzeit-Bild des zu operierenden Objekts zu erzeugen, das Informationen über die Aussenkontur und innere Struktur des zu operierenden Objekts umfasst.

Vorzugsweise ist die röntgenbasierten Detektionseinrichtung möglichst schwach strahlend bzw. niedrig dosiert, um die Strahlenbelastung für den Patienten und das Operationspersonal so gering wie möglich zu halten.

Die gemäss der Erfindung vorgesehene Bildanzeigeeinrichtung zur Darstellung des virtuellen Echtzeit-Bildes kann beispielsweise als stationärer Monitor im Operationssaal ausgebildet sein. Denkbar ist aber auch, dass die Bildanzeigeeinrichtung als ein tragbarer Monitor ausgebildet ist, der insbesondere am Handgelenk des Operateurs oder am Kopf vor den Augen des Operateurs oder an einem Instrument/Werkzeug angeordnet werden kann.

Weitere Einzelheiten der Erfindung und insbesondere eine beispielhafte Ausführungsform der vorgeschlagenen Vorrichtung werden im Folgenden anhand der beigefügten Zeichnungen erläutert.

In den Zeichnungen zeigen:
- Fig. 1: eine schematische Ansicht eines möglichen Ausführungsbeispiels der erfindungsgemässen Vorrichtung,
- Fig. 2: eine Detailansicht der erfindungsgemässen Vorrichtung gemäss Fig. 1, und
- Fig. 3: eine schematische Ansicht einer weiteren möglichen Anordnung der erfindungsgemässen Vorrichtung.

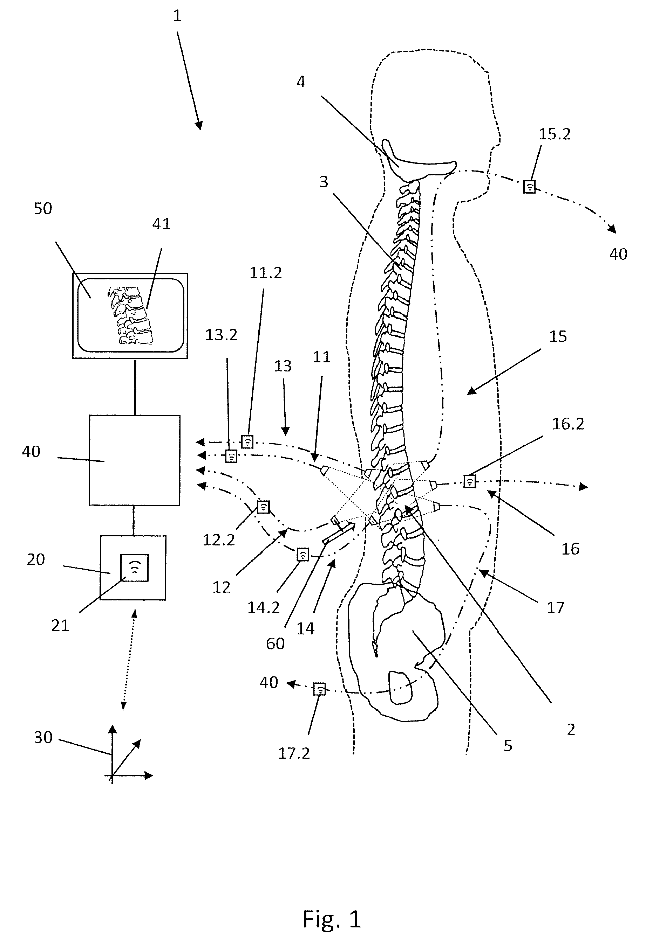

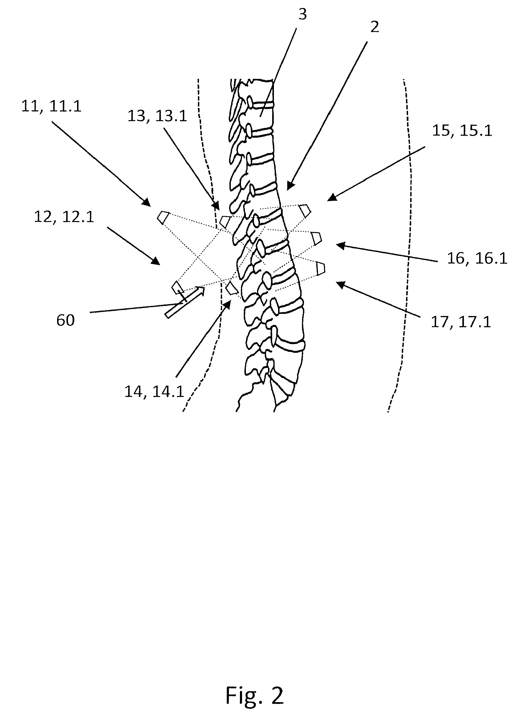

Die Fig. 1 und 2 zeigen ein mögliches Ausführungsbeispiel der erfindungsgemässen Vorrichtung für die intraoperative, bildgesteuerte Navigation bei chirurgischen Eingriffen im Bereich der Wirbelsäule 3 und/oder im daran angrenzenden Thorax-, Becken- 5 oder Kopfbereich 4.

Die Vorrichtung 1 umfasst mehrere nicht-röntgenbasierte Detektionseinrichtungen 11-17, die jeweils dazu ausgebildet sind, um ein zu operierenden Objekt 2 an der Wirbelsäule 3 eines Patienten verteilt angeordnet zu werden. Jede dieser Detektionseinrichtungen 11-17 ist ferner dazu ausgebildet, intraoperativ jeweils zumindest die Aussenkontur wenigstens eines Teilbereichs des zu operierenden Objekts 2 sowie die Lage dieses Teilbereichs relativ zur jeweiligen Detektionseinrichtung 11-17 in Echtzeit zu erfassen.

Wie den Fig. 1 und 2 zu entnehmen ist, sind die Detektionseinrichtungen 11 und 12 bzw. die ihnen zugeordneten Detektionsmittel 11.1 und 12.1 ausserhalb des Körpers angeordnet, während die Detektionseinrichtungen 13-17 bzw. die ihnen zugeordneten Detektionsmittel 13.1-17.1 innerhalb des Körpers angeordnet sind. Im vorliegenden Ausführungsbeispiel sind die Detektionseinrichtungen 13 und 14 bzw. die ihnen zugeordneten Detektionsmittel 13.1 und 14.1 operativ in das paravertebrale Muskelgewebe um den zu operierenden freipräparierten Knochenbereich 2 eingebracht. Ebenso ist das Detektionsmittel 16.1 der Detektionseinrichtung 16 operativ über die Bauchhöhle an die Vorderseite des zu operierenden Knochenbereichs 2 der Wirbelsäule 3 eingebracht.

Demgegenüber ist das Detektionsmittel 15.1 der Detektionseinrichtung 15 als Endoskop ausgebildet und über den Mund, den Rachenraum und die Speiseröhre in den Magen eingebracht, um von dort aus die Aussenkontur eines Teilbereichs des zu operierenden Knochenbereichs 2 aus Richtungen des Magens oder der Speiseröhre zu erfassen. In ähnlicher Weise ist auch das Detektionsmittel 17.1 der Detektionseinrichtung 17 als Endoskop ausgebildet und rektal in den Dickdarm eingebracht, um von dort aus die Aussenkontur eines anderen Teilbereichs des zu operierenden Knochenbereichs 2 aus dem Körperinneren heraus zu erfassen.

Vorzugsweise sind im vorliegenden Ausführungsbeispiel alle innerhalb des Körpers eingebrachten Detektionseinrichtungen 13 bis 17 bzw. die ihnen zugeordneten Detektionsmittel 13.1-17.1 als ultraschallbasierte Detektionseinrichtungen bzw. Detektionsmittel ausgebildet. Die beiden Detektionsmittel 13.1 und 14.1 sind im vorliegenden Ausführungsbeispiel als sogenannte Minisonden-Systeme ausgebildet, die durch einen jeweiligen Biopsiekanal in das paravertebrale Muskelgewebe eingeschoben werden können. Demgegenüber sind die Detektionsmittel 15.1 und 17.1 als endoskopische Ultraschallsonden ausgebildet.

Die beiden ausserhalb des Körpers angeordneten Detektionseinrichtungen 11 und 12 bzw. die ihnen zugeordneten Detektionsmittel 11.1 und 12.1 sind im vorliegenden Ausführungsbeispiel als optische Konturscanner ausgebildet, die jeweils einen Teilbereich der Aussenkontur des zu operierenden Knochenbereichs 2 optisch mittels Laserlicht abtasten.

Die in den Fig. 1 und 2 gestrichelt gezeichneten Linien, die von den jeweiligen Detektionsmitteln 11.1-17.1 ausgehen, repräsentieren jeweils das entsprechende Detektionsfeld der jeweiligen Detektionseinrichtung 11-17. Wie zu erkennen ist, können sich die Detektionsfelder verschiedener Detektionseinrichtungen 11-17 überlappen. Im vorliegenden Ausführungsbeispiel überlappen sich die Detektionsfelder insbesondere so, dass eine nahezu vollständige Rundum-Darstellung des zu operierenden Knochenbereichs 2 ermöglicht wird.

Alle Detektionseinrichtungen 11-17 sind ferner dazu ausgebildet, die Lage des jeweils erfassten Teilbereichs zu sich selbst zu erfassen. Hierzu eignen sich insbesondere ultraschallbasierte Detektionseinrichtungen und optische Konturscanner. Dies ist eine Grundvoraussetzung dafür, die erfassten Aussenkonturdaten überhaupt in Bezug zu einem stationären Bezugssystem zu setzen.

Um die jeweiligen Lagen/Positionen der Detektionseinrichtungen 11-17 in Bezug auf ein stationäres Bezugssystem 30, etwa ein bezüglich des Operationssaals stationäres Bezugssystem 30, zu ermitteln, weist die erfindungsgemässe Vorrichtung 1 ferner eine Positionsbestimmungseinrichtung 20 auf. Mit der Lage/Position der Detektionseinrichtungen 11-17 ist vornehmlich die Lage/Position der jeweiligen Detektionsmittel 11.1-17.1 in Bezug auf das stationäre Bezugssystem 30 gemeint.

Im vorliegenden Ausführungsbeispiel ist die Positionsbestimmungseinrichtung 20 als elektromagnetisches Positionserkennungssystem ausgebildet. Dieses System umfasst einen an jedem der Detektionseinrichtungen passiven Positionsmarker 11.2, 12.2 oder einen Positionssender 13.2-17.2, der über eine entfernt angeordnete, kombinierte Positionsmarker-Erkennungseinrichtung und Positionssender-Empfängereinrichtung 21 überwacht wird. Diese kombinierte Einrichtung ist dazu ausgebildet, in Echtzeit einerseits die räumliche Position der Positionsmarker 11.2 und 12.2 und damit die räumliche Position der Detektionseinrichtungen 11 und 12 zu erkennen und zu verfolgen und andererseits die von den Positionssendern 13.2-17.2 ausgesandten Positionssignale zu empfangen und daraus eine räumliche Position der zu geordneten Detektionseinrichtungen 13-17 zu bestimmen.

Für die Erkennung müssen die Positionsmarker 11.2 und 12.2 typischerweise ausserhalb des Körpers angeordnet sein. Demgegenüber können die Positionssender 13.2-17.2 sowohl innerhalb als auch ausserhalb des Körpers angeordnet sein. Im vorliegenden Ausführungsbeispiel ist insbesondere der Positionssender 16.2 dazu ausgebildet, innerhalb des Körpers angeordnet zu werden und von dort aus Positionssignale, die Informationen über die Lage des Detektionsmittel 16.1, nach aussen zu senden, so dass sie dort von der Positionssender-Empfängereinrichtung 21 empfangen werden können. Im vorliegenden Ausführungsbeispiel sind sämtliche Positionssender 13.2-17.2 dazu ausgerichtet, drahtlos die jeweiligen Positionssignale an die Positionssender-Empfängereinrichtung 21 zu übertragen. Denkbar ist aber auch, dass die Übertragung der Positionssignale bei einem oder mehreren der Positionssender drahtgebunden erfolgt.

Die von der Positionsbestimmungseinrichtung 20 bezüglich des stationären Bezugssystems 30 ermittelten Lagedaten der Detektionseinrichtungen 13-17 bzw. der Detektionsmittel 13.1-17.1 sowie die von den Detektionsmitteln 13.1-17.1 bzw. den Detektionseinrichtungen 13-17 ermittelten Lagedaten relativ zum aussenkonturmässig erfassten Teilbereichs des zu operierenden Knochenbereichs 2 werden einer Datenverarbeitungseinrichtung 40 zur Verfügung gestellt.

Ferner übermitteln die Detektionseinrichtungen 13-17 an die Datenverarbeitungseinrichtung 40 jeweils Bilddaten, die die entsprechenden, von ihnen in Echtzeit erfassten, Aussenkonturen bzw. Aussenkonturdaten des jeweiligen Teilbereichs des zu operierenden Knochenbereichs 2 repräsentieren. Zum Zweck dieses Datentransfers sind die Detektionseinrichtungen 13-17 sowie die Positionsbestimmungseinrichtung 20 mit der Verarbeitungseinrichtung 40 wirkverbunden. Dieser Datentransfer kann beispielsweise über eine drahtgebundene Datenverbindung oder über eine drahtlose Datenverbindung erfolgen.

Die Datenverarbeitungseinrichtung 40 ist im Weiteren dazu ausgebildet, auf Basis der übermittelten Bild- und Lagedaten ein auf das stationäre Bezugssystem 30 referenziertes, virtuelles, vorzugsweise dreidimensionales Echtzeit-Bild 41 des zu operierenden Knochenbereichs zu erzeugen, das aus den einzelnen Bilddatensätzen der jeweiligen Teilbereiche zusammengesetzt ist. Dieses virtuelle Echtzeit-Bild beruht in erfindungsgemässer Weise vollständig auf tatsächlichen Echtzeit-Bilddaten, die nicht nur die Nachverfolgung der Echtzeit-Position des Operationsbereichs relativ zum stationären Bezugssystem 30 erlauben, sondern auch in Echtzeit wenigstens die tatsächliche Aussenkontur des zu operierenden Knochenbereichs 2 quasi in situ bildlich wiedergeben. Aufgrund der mehreren innerhalb des Körpers angeordneten Detektionseinrichtungen 13-17 kann in erfindungsgemässer Weise ein im Vergleich zum Stand der Technik wesentlich umfassenderes Bild des zu operierenden Knochenbereichs erzeugt werden. Aufgrund der in Richtung Rückenseite erfassenden Detektionseinrichtungen 15-17 können insbesondere Informationen über die Aussenkontur zumindest eines dem Körperinneren zugewandten Teilbereichs des zu operierenden Knochenbereichs enthalten werden. Im Idealfall kann sogar ein virtuelles Echtzeit-Rundumbild der Aussenkontur des zu operierenden Knochenbereichs erzeugt werden, wodurch die Navigationsgenauigkeit und das Sicherheitsgefühl für den Operateur erheblich verbessert wird.

Das von der Verarbeitungseinrichtung 40 erzeugte virtuelle Echtzeit-Bild 41 kann auf einer damit wirkverbundenen Bildanzeigeeinrichtung 50 dargestellt werden. In vorteilhafter Weise ist diese Bildanzeigeeinrichtung 50 im vorliegenden Ausführungsbeispiel als tragbarer Monitor bzw. als tragbares Display ausgebildet, das insbesondere am Handgelenk des Operateurs oder in Art einer Monitorbrille vor den Augen des Operateurs oder an einem Operationswerkzeug/-instrument befestigt werden kann.

Im vorliegenden Ausführungsbeispiel umfasst die erfindungsgemässe Vorrichtung 1 zudem wenigstens ein Operationswerkzeug 60, das - ähnlich wie die Detektionseinrichtungen 13-17 - mit einem Positionssender oder einem Positionsmarker ausgestattet ist, mit dessen Hilfe die räumliche Lage des Operationswerkzeuges 60 relativ zum stationären Bezugssystem 30 und damit zum zu operierenden Knochenbereich 2 bestimmt werden kann. Zu diesem Zwecke ist die Positionsbestimmungseinrichtung 20 ferner zur Ermittlung der Lage des wenigstens einen Operationswerkzeuges relativ zum stationären Bezugsystem 30 ausgebildet. Ferner kann die Verarbeitungseinheit 40 zum Einblenden eines Indikators in das virtuelle Echtzeit-Bild 41 ausgebildet sein, der das wenigstens eine Operationswerkzeug 60 und dessen Lage relativ zum stationären Bezugsystem 30 repräsentiert. Hierdurch werden zusätzlich die Navigationsgenauigkeit und das Sicherheitsgefühl für den Operateur verbessert.

Wie insbesondere in Fig. 2 gezeigt ist, kann es vorgesehen sein, dass wenigstens eine der Detektionseinrichtungen - im vorliegenden Ausführungsbeispiel die Detektionseinrichtung 12 - an dem Operationswerkzeug 60 angeordnet ist. Hierzu ist das Operationswerkzeug 60 im Speziellen mit einer Halterung ausgebildet, um die Detektionseinrichtung 12 daran zu befestigen.

Um die Funktionalität der Vorrichtung gegen etwaige äussere Störeinflüsse abzusichern und somit das Sicherheitsgefühl für den Operateur konstant aufrecht zu erhalten, ist die Verarbeitungseinrichtung 40 ferner dazu ausgebildet, bei einem temporären Ausfall der Bilderfassung und/oder der Lage-/Positionsermittlung einer Detektionseinrichtung 11-17 den von dieser Detektionseinrichtung 11-17 erfassten Teilbereich des zu operierenden Knochenbereichs 2 in dem virtuellen Echtzeit-Bild 41 auf Basis der zu einem früheren Zeitpunkt von dieser Detektionseinrichtung 11-17 bei funktionierender Lageermittlung erfassten Bilddaten zu erzeugen. Dazu kann es insbesondere vorgesehen sein, dass die zu einem früheren Zeitpunkt erfassten Bilddaten des entsprechenden Teilbereichs in Echtzeit an die aktuelle Lage der von den übrigen Detektionseinrichtungen 11-17 erfassten Teilbereiche anpasst werden.

Für den Fall, dass lediglich die Lage-/Positionsermittlung für eine oder mehrere der Detektionseinrichtungen 11-17 temporär ausfällt, nicht jedoch die Bilderfassung der betroffenen Detektionseinrichtung(en) 11-17 selbst, kann es ferner vorgesehen sein, den von der jeweils betroffenen Detektionseinrichtung 11-17 erfassten Teilbereich des zu operierenden Knochenbereichs 2 in dem virtuellen Echtzeit-Bild 41 auf Basis der aktuell erfassten Bilddaten zu erzeugen. Hierzu werden die aktuell erfassten Bilddaten des entsprechenden Teilbereichs im Hinblick auf ihre Lage relativ zum stationären Bezugssystem 41 in Echtzeit an die aktuelle Lage der von den übrigen Detektionseinrichtungen erfassten Teilbereiche angepasst.

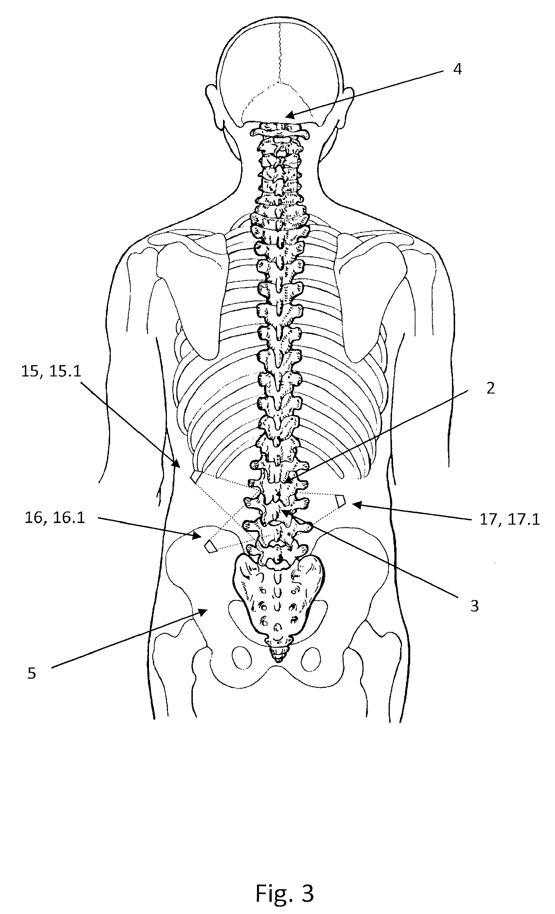

Fig. 3 zeigt eine weitere mögliche Anordnung von Detektionseinrichtungen 15-17 bzw. zugeordneten Detektionsmitteln 15.1-17.1 der erfindungsgemässen Vorrichtung innerhalb des Körpers. Gemäss dieser beispielhaften Ausgestaltung sind wenigstens die Detektionsmittel 15.1-17.1 von bespielhaft drei Detektionseinrichtungen 15-17 seitlich links bzw. rechts an der Wirbelsäule 3 angeordnet und so ausgerichtet, dass sie die Aussenkontur derjenigen Bereiche des zu operierenden Knochenbereichs 2 erfassen, die in distaler Richtung der linken und rechten Körperseite zugewandt sind. Selbstverständlich kann die Anordnung der Detektionseinrichtungen 15-17 gemäss Fig. 3 um weitere Detektionseinrichtungen, die innerhalb oder ausserhalb des Körpers angeordnet werden können, ergänzt werden. Insbesondere kann die spezielle Anordnung der Detektionseinrichtungen gemäss Fig. 3 auch mit jener Anordnung von Detektionseinrichtungen gemäss Fig. 1 und 2 kombiniert werden. Im Idealfall kann mit einer entsprechenden "Rundum-Anordnungen von Detektionseinrichtungen innerhalb und ausserhalb des Körpers sogar ein virtuelles Echtzeit-Rundumbild der Aussenkontur des zu operierenden Knochenbereichs erzeugt werden, wodurch die Navigationsgenauigkeit und das Sicherheitsgefühl für den Operateur erheblich verbessert wird.

## Patentansprüche

1. Vorrichtung (1) für die intraoperative, bildgesteuerte Navigation bei chirurgischen Eingriffen im Bereich der Wirbelsäule (3) und/oder im daran angrenzenden Thorax-, Becken- (5) oder Kopfbereich (4), umfassend
mehrere nicht-röntgenbasierte Detektionseinrichtungen (11-17), die jeweils dazu ausgebildet sind, verteilt um wenigstens ein zu operierendes Objekt (2) im Bereich der Wirbelsäule (3) und/oder im daran angrenzenden Thorax, Becken- oder Kopfbereich angeordnet zu werden und intraoperativ in Echtzeit Bild- und Lagedaten zu erfassen, die jeweils Informationen über zumindest die Aussenkontur wenigstens eines Teilbereichs des zu operierenden Objekts (2) sowie über die Lage des Teilbereichs relativ zur jeweiligen Detektionseinrichtung (11-17) umfassen,
eine Positionsbestimmungseinrichtung (20), die dazu ausgebildet ist, in Echtzeit Daten zur jeweiligen Position der Detektionseinrichtungen (11-17) relativ zu einem stationären Bezugssystem (30) zu ermitteln,
eine mit den Detektionseinrichtungen (11-17) und der Positionsbestimmungseinrichtung (20) wirkverbundene Datenverarbeitungseinrichtung (40), die dazu ausgebildet ist, auf Basis der jeweiligen Bild- und Lagedaten der Detektionseinrichtungen (11-17) und der jeweiligen Positionsdaten der Positionsbestimmungseinrichtung (20) ein auf das stationäre Bezugssystem (30) referenziertes, virtuelles, vorzugsweise dreidimensionales Echtzeit-Bild (41) des zu operierenden Objekts (2) zu erzeugen,
eine mit der Datenverarbeitungseinrichtung (40) wirkverbundene Bildanzeigeeinrichtung (50) zur Darstellung des virtuellen Echtzeit-Bildes (41),
**dadurch gekennzeichnet, dass** wenigstens eine der mehreren Detektionseinrichtungen (13-17) wenigstens ein Detektionsmittel (13.1-17.1) aufweist, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts (2) umfassen.

2. Vorrichtung (1) nach Anspruch 1, **dadurch gekennzeichnet, dass** die Datenverarbeitungseinrichtung (40) dazu ausgebildet ist, präoperativ gewonnene Bilddaten des zu operierenden Objekts mit den Bilddaten der Detektionseinrichtungen (13-17) zu überlagern, um auf Basis der mit den Bilddaten der Detektionseinrichtungen (13-17) überlagerten, präoperativ gewonnenen Bilddaten das virtuelle, auf das stationäre Bezugssystem referenzierte Echtzeit-Bild des zu operierenden Objekts zu erzeugen.

3. Vorrichtung (1) nach Anspruch 1 oder 2, **dadurch gekennzeichnet, dass** die wenigstens eine Detektionseinrichtung (13-17) mit dem wenigstens einen im Inneren des Körpers anordenbaren Detektionsmittel (13.1-17.1) eine ultraschallbasierte Detektionseinrichtung oder eine auf Terahertzstrahlung basierte Detektionseinrichtung ist.

4. Vorrichtung (1) nach einem der vorhergehenden Ansprüche, **dadurch gekennzeichnet, dass** das wenigstens eine im Inneren des Körpers anordenbare Detektionsmittel (13.1-17.1) der wenigstens einen Detektionseinrichtung (13-17) dazu ausgebbildet ist, über eine präformierte Körperhöhle, insbesondere die Nase, den Mund, den Rachenraum, die Speiseröhre, die Luftröhre, den Magen-Darmtrakt, die Harnblase, oder über ein Blutgefäss in den Körper eingebracht zu werden.

5. Vorrichtung (1) nach einem der vorhergehenden Ansprüche, **dadurch gekennzeichnet, dass** das wenigstens eine im Inneren des Körpers anordenbare Detektionsmittel (13.1-17.1) der wenigstens einen Detektionseinrichtung (13-17) dazu ausgebbildet ist, operativ in ein Gewebe, beispielsweise in paravertebrales Muskelgewebe oder Muskelgewebe des Gesässmuskels, und/oder in Fettgewebe, beispielsweise den Epiduralraum, eingebracht zu werden.

6. Vorrichtung (1) nach einem der vorhergehenden Ansprüche, **dadurch gekennzeichnet, dass** wenigstens eine der mehreren Detektionseinrichtungen (11, 12) und/oder wenigstens ein Detektionsmittel (11.1, 12.1) wenigstens einer der mehreren Detektionseinrichtungen (11, 12) dazu ausgebildet ist, ausserhalb des Körpers angeordnet zu werden, um Bilddaten zu erfassen, die Informationen über zumindest die Aussenkontur eines dem Körperäusseren zugewandten Teilbereichs des zu operierenden Objekts (2) umfassen.

7. Vorrichtung (1) nach Anspruch 6, **dadurch gekennzeichnet, dass** die wenigstens eine ausserhalb des Körpers anordenbare Detektionseinrichtung (11, 12) und/oder das wenigstens eine ausserhalb des Körpers anordenbare Detektionsmittel (11.1, 12.1) eine ultraschallbasierte Detektionseinrichtung oder ein optischer Konturscanner oder eine auf Terahertzstrahlung basierte Detektionseinrichtung ist.

8. Vorrichtung (1) nach einem der vorhergehenden Ansprüche, **dadurch gekennzeichnet, dass** die Vorrichtung (1) wenigstens ein Operationswerkzeug (60) umfasst, wobei die Positionsbestimmungseinrichtung (20) ferner dazu ausgebildet ist, in Echtzeit Daten zur Position des wenigstens einen Operationswerkzeuges (60) relativ zum stationären Bezugsystem (30) zu ermitteln, und wobei die Datenverarbeitungseinheit (40) ferner zum Einblenden eines das Operationswerkzeug (60) und dessen Lage repräsentierenden Indikators in das virtuelle Echtzeit-Bild (41) ausgebildet ist.

9. Vorrichtung nach einem der vorhergehenden Ansprüche, insbesondere nach Anspruch 8, **dadurch gekennzeichnet, dass** wenigstens eine der mehreren Detektionseinrichtungen (11-17) und/oder wenigstens ein Detektionsmittel (11.1-17.1) wenigstens einer der mehreren Detektionseinrichtungen (11-17) dazu ausgebildet ist, an einem Operationswerkzeug (60) angeordnet zu werden, insbesondere dass wenigstens eine der mehreren Detektionseinrichtungen (11-17) und/oder wenigstens ein Detektionsmittel (11.1-17.1) wenigstens einer der mehreren Detektionseinrichtungen (11-17) an dem Operationswerkzeug (60) angeordnet ist.

10. Vorrichtung (1) nach einem der Ansprüche 1 bis 9, **dadurch gekennzeichnet, dass** die Datenverarbeitungseinrichtung (40) dazu ausgebildet ist, bei einem temporären Ausfall der Bilddaten-, der Lagedaten und/oder der Positionsdatenermittlung wenigstens einer der Detektionseinrichtungen (11-17) den von dieser Detektionseinrichtung (11-17) erfassten Teilbereich des zu operierenden Objekts (2) in dem virtuellen Echtzeit-Bild (41) auf Basis der zu einem früheren Zeitpunkt von dieser Detektionseinrichtung (11-17) bei funktionierender Lagedaten- und Positionsdatenermittlung erfassten Bilddaten zu erzeugen, wobei die zu einem früheren Zeitpunkt erfassten Bilddaten des entsprechenden Teilbereichs in Echtzeit an die aktuelle Lage der von den übrigen Detektionseinrichtungen (11-17) erfassten Teilbereiche anpasst werden.

11. Vorrichtung (1) nach einem der Ansprüche 1 bis 9, **dadurch gekennzeichnet, dass** die Verarbeitungseinrichtung (40) dazu ausgebildet ist, bei einem temporären Ausfall der Lagedaten- und/oder Positionsdatenermittlung einer Detektionseinrichtung (11-17) den von dieser Detektionseinrichtung (11-17) erfassten Teilbereich des zu operierenden Objekts (2) in dem virtuellen Echtzeit-Bild (41) auf Basis der aktuell erfassten Bilddaten zu erzeugen, wobei die aktuell erfassten Bilddaten des entsprechenden Teilbereichs in Echtzeit an die aktuelle Lage der von den übrigen Detektionseinrichtungen (11-17) erfassten Teilbereiche anpasst werden.

12. Vorrichtung (1) nach einem der Ansprüche 1 bis 11, **dadurch gekennzeichnet, dass** die Vorrichtung (1) ferner wenigstens eine röntgenbasierte Detektionseinrichtung aufweist, die dazu ausgebildet ist, intraoperativ in Echtzeit Bilddaten zu erfassen, die Informationen über die Aussenkontur und innere Struktur wenigstens eines Teilbereichs des zu operierenden Objekts (2) umfassen, wobei die Datenverarbeitungseinrichtung (40) mit der röntgenbasierten Detektionseinrichtung wirkverbunden ist und dazu ausgebildet ist, die Bilddaten der röntgenbasierten Detektionseinrichtung mit den Bilddaten der nicht-röntgenbasierten Detektionseinrichtungen (13-17) zu überlagern, um auf Basis der miteinander überlagerten Bilddaten das virtuelle auf das stationäre Bezugssystem referenzierte Echtzeit-Bild des zu operierenden Objekts zu erzeugen, das Informationen über die Aussenkontur und innere Struktur des zu operierenden Objekts umfasst.

13. Vorrichtung (1) nach einem der Ansprüche 1 bis 11, **dadurch gekennzeichnet, dass** die Vorrichtung (1) ferner wenigstens eine röntgenbasierte Detektionseinrichtung aufweist, die dazu ausgebildet ist, intraoperativ in Echtzeit Bild- und Lagedaten zu erfassen, die Informationen über die Aussenkontur und innere Struktur wenigstens eines Teilbereichs des zu operierenden Objekts (2) sowie über die Lage des Teilbereichs relativ zur röntgenbasierten Detektionseinrichtung umfassen, wobei die Positionsbestimmungseinrichtung (20) ferner dazu ausgebildet ist, in Echtzeit Daten zur Position der röntgenbasierten Detektionseinrichtung relativ zum stationären Bezugssystem (30) zu ermitteln, und wobei die Datenverarbeitungseinrichtung (40) ferner mit der röntgenbasierten Detektionseinrichtung wirkverbunden ist und dazu ausgebildet ist, auf Basis der jeweiligen Bild- und Lagedaten der nicht-röntgenbasierten Detektionseinrichtungen (11-17), der Bild- und Lagedaten der röntgenbasierten Detektionseinrichtung und der jeweiligen Positionsdaten der Positionsbestimmungseinrichtung (20) ein auf das stationäre Bezugssystem (30) referenziertes, virtuelles, vorzugsweise dreidimensionales Echtzeit-Bild (41) des zu operierenden Objekts (2) zu erzeugen, das Informationen über die Aussenkontur und innere Struktur des zu operierenden Objekts umfasst.

14. Vorrichtung nach einem der vorhergehenden Ansprüche, **dadurch gekennzeichnet, dass** die Detektionseinrichtungen (13-17) dazu ausgebildet sind, miteinander zu kommunizieren, insbesondere Informationen über ihre jeweilige Position und/oder Lage, vorzugsweise relativ zum stationären Bezugssystem (30), auszutauschen.

15. Vorrichtung nach einem der vorhergehenden Ansprüche, **dadurch gekennzeichnet, dass** wenigstens eine der mehreren Detektionseinrichtungen (13-17), insbesondere wenigstens ein Detektionsmittel (13.1-17.1) wenigstens einer der Detektionseinrichtungen (13-17), dazu ausgebildet ist, die Position und/oder Lage wenigstens einer anderen Detektionseinrichtung (13-17) bzw. die Position und/oder Lage des jeweiligen Detektionsmittels (13.1-17.1) der wenigstens einen anderen Detektionseinrichtung (13-17) relativ zu sich, und vorzugsweise dadurch auch über die Positionsbestimmungseinrichtung (20) relativ zum stationären Bezugssystem (30), zu ermitteln.

16. Vorrichtung für die intraoperative, bildgesteuerte Navigation bei chirurgischen Eingriffen im Bereich der Wirbelsäule und/oder im daran angrenzenden Thorax-, Becken- oder Kopfbereich vorgeschlagen, umfassend
wenigstens eine Detektionseinrichtung, die dazu ausgebildet ist, intraoperativ in Echtzeit Bild- und Lagedaten zu erfassen, die Informationen über zumindest die Aussenkontur wenigstens eines Teilbereichs eines zu operierenden Objekts sowie über die Lage des Teilbereichs relativ zur röntgenbasierten Detektionseinrichtung umfassen,
eine Positionsbestimmungseinrichtung, die dazu ausgebildet ist, in Echtzeit Daten zur Position der wenigstens einen Detektionseinrichtung relativ zu einem stationären Bezugssystem zu ermitteln,
eine mit der wenigstens einen Detektionseinrichtung und der Positionsbestimmungseinrichtung wirkverbundene Datenverarbeitungseinrichtung, die dazu ausgebildet ist, präoperativ gewonnene Bilddaten des zu operierenden Objekts mit den Bilddaten der wenigstens einen Detektionseinrichtung zu überlagern, um auf Basis der mit den Bilddaten der Detektionseinrichtung überlagerten, präoperativ gewonnenen Bilddaten ein virtuelles auf das stationäre Bezugssystem referenzierte Echtzeit-Bild des zu operierenden Objekts zu erzeugen, und
eine mit der Datenverarbeitungseinrichtung wirkverbundene Bildanzeigeeinrichtung zur Darstellung des virtuellen Echtzeit-Bildes,
**dadurch gekennzeichnet, dass** die wenigstens eine Detektionseinrichtung wenigstens ein Detektionsmittel aufweist, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen.

17. Vorrichtung nach Anspruch 16, **dadurch gekennzeichnet, dass** die wenigstens eine Detektionseinrichtung mit dem wenigstens einen im Inneren des Körpers anordenbaren Detektionsmittel eine ultraschallbasierte Detektionseinrichtung oder eine auf Terahertzstrahlung basierte Detektionseinrichtung ist.

18. Vorrichtung nach Anspruch 16 oder 17, **dadurch gekennzeichnet, dass** das wenigstens eine im Inneren des Körpers anordenbare Detektionsmittel der wenigstens einen Detektionseinrichtung dazu ausgebbildet ist, über eine präformierte Körperhöhle, insbesondere die Nase, den Mund, den Rachenraum, die Speiseröhre, die Luftröhre, den Magen-Darmtrakt, die Harnblase, oder über ein Blutgefäss in den Körper eingebracht zu werden.

19. Vorrichtung nach einem der Ansprüche 16 - 18, **dadurch gekennzeichnet, dass** das wenigstens eine im Inneren des Körpers anordenbare Detektionsmittel der wenigstens einen Detektionseinrichtung dazu ausgebbildet ist, operativ in ein Gewebe, beispielsweise in paravertebrales Muskelgewebe oder Muskelgewebe des Gesässmuskels, und/oder in Fettgewebe, beispielsweise den Epiduralraum, eingebracht zu werden.

20. Vorrichtung nach einem der Ansprüche 16 - 19, **dadurch gekennzeichnet, dass** die Vorrichtung wenigstens ein Operationswerkzeug umfasst, wobei die Positionsbestimmungseinrichtung ferner dazu ausgebildet ist, in Echtzeit Daten zur Position des wenigstens einen Operationswerkzeuges relativ zum stationären Bezugsystem zu ermitteln, und wobei die Datenverarbeitungseinheit ferner zum Einblenden eines das Operationswerkzeug und dessen Lage repräsentierenden Indikators in das virtuelle Echtzeit-Bild ausgebildet ist.
